# EUROPEAN PATENT APPLICATION

(11) **EP 3 476 357 A1**
(43) Date of publication of application: **01.05.2019**
(21) Application number: 17197978.4
(22) Date of filing: 24.10.2017
(51) Int. Cl.: A61B 34/20, A61C 1/08, A61B 90/00, A61C 8/00, A61C 1/00, A61B 17/16, A61B 17/17, G06F 1/16, A61B 34/10, A61B 34/00

(54) **AN OPERATIONAL SYSTEM ON A WORKPIECE AND METHOD THEREOF**

(71) Applicant: GuideMia Biotechnologies (Shanghai) Ltd., Shanghai (CN)
(72) Inventor: GAO, Fei, Buena Park, California 90620 (US)
(74) Representative: London IP Ltd

(57) **Abstract**

The present invention provides an ergonomic operational system (100) and method thereof for a human operator (10) such as a dental surgeon in image guided implantation. A physical or virtual display (52) in close proximity to, or integrated with, the dental drill (30) shows the information that demands a high frequency of observation from the surgeon. The surgeon is thus able to monitor the drilling site and the display at the same time, without moving his head toward other display (56) not within his field of view. The invention exhibits numerous technical merits such as simplicity of operation, improved operational safety, higher productivity, and enhanced efficiency, among others.

## Description

### FIELD OF THE INVENTION

The present invention generally relates to an operational system or an apparatus for a human operator to operate on a workpiece, and a method thereof. Although the invention will be illustrated, explained and exemplified by image guided drilling of a patient's jawbone, it should be appreciated that the present invention can also be applied to other fields, for example, image-guided industrial procedures; other image-guided surgical procedures such as surgery within the ear, nose, throat, and paranasal sinuses; image guided implantation or installation of a hearing aid; image-guided delivery of therapeutics e.g. to an eye or other organs; image guided catheters; image-guided radiotherapy for e.g. treatment of a tumor; image-guided heart valve placement or repair; and the like.

### BACKGROUND OF THE INVENTION

Titanium implantation is widely used for restoring a lost tooth. Drilling the patient's jawbone to prepare an implant site is an important, but very risky, step in the entire procedure. The surgeon must be very cautious to avoid injury to the patient. Examples of such potential damage include inadvertent entry into the mandibular nerve canal, possible perforation of the cortical plates, or damage to adjacent teeth. This requires the surgeon to closely and constantly monitor the dynamic spatial relationship between the drill bit and the jawbone, in order to execute a well-calculated drilling plan.

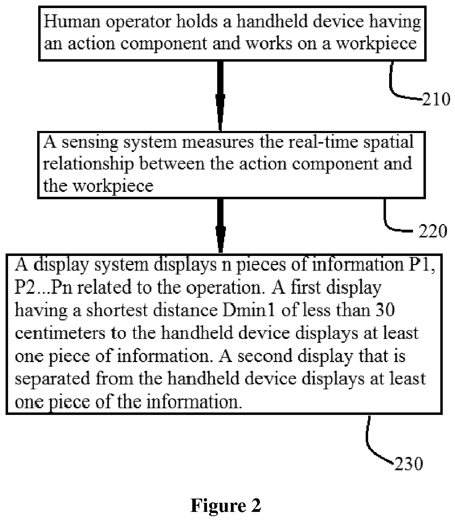

In an image guided drilling process, a big-screen display is placed in the surgical room. The display shows, in real time, the location of a drill bit mounted onto a handpiece in relationship to the 3D image of a patient's jawbone overlaid on a planned drilling trajectory. The surgeon is guided by the display during the drilling of the jawbone. For example, U.S. Patent Application Publication 20080171305 by Sonenfeld et al. illustrates such an implant surgery as shown in its FIG. 2J. A challenge for the surgeon is that, while he focus on the display, he must also keep an eye on the patient's jawbone in real world for safety. Therefore, the surgeon has to frequently move his head up and down to obverse both the drilling site in the real world and the virtual drill bit and jawbone on the display, while he is drilling the real jawbone. This rigorous requirement makes the surgeon feel nervous and stressful, and increases the likelihood of misoperation, which may result in an irreparable damage on the patient's jawbone, or a poor execution of the drilling plan.

Therefore, there exists a need to overcome the aforementioned problems. Advantageously, the present invention provides an operational system or an apparatus for a human operator to operate on a workpiece, which exhibits numerous technical merits such as user-friendly and ergonomic design, simplicity of operation, improved operational safety, higher productivity, and enhanced efficiency, among others.

### SUMMARY OF THE INVENTION

One aspect of the present invention provides an operational system or an apparatus for a human operator to operate on a workpiece. The system includes:
(1) a handheld device for the human operator to hold in hand, wherein the handheld device includes an action component that works on the workpiece under the control of the human operator;
(2) a sensing system (e.g. 3D camera, 3D sensor head, or 3D tracking system) that measures the spatial relationship between the action component and the workpiece;
(3) a display system for displaying n pieces of information P1, P2...Pn which are selected from the working conditions of the handheld device, the conditions of the workpiece, the 3D image of the handheld device, the 3D image of the workpiece, a real-time spatial relationship between the action component and the workpiece, and a preplanned spatial relationship between the action component and the workpiece, wherein n is an integer and n ≥2. The display system comprises a first display that has a shortest distance Dmin1 of less than 30 centimeters to the handheld device. For example, when the first display is attached to, or integrated with, the handheld device, Dmin = 0. The display system further comprises a second display that is separated from the handheld device. The first display may be a physical display (or a hardware display) or a virtual display that displays at least one piece of information selected from said n pieces of information; and the second display displays at least one piece of information selected from said n pieces of information. The two "at least one piece of information" may be the same or different.

Another aspect of the invention provides a method of operating on a workpiece comprising:
(i) providing a handheld device for a human operator to hold in hand, wherein the handheld device includes an action component that works on the workpiece under the control of the human operator;
(ii) measuring the spatial relationship between the action component and the workpiece with a sensing system;
(iii) providing a display system for displaying n pieces of information P1, P2...Pn which are selected from the working conditions of the handheld device, the conditions of the workpiece, the 3D image of the handheld device, the 3D image of the workpiece, a real-time spatial relationship between the action component and the workpiece, and a preplanned spatial relationship between the action component and the workpiece, wherein n is an integer and n ≥2; and wherein the display system comprises a first display that has a shortest distance Dmin1 of less than 30 centimeters to the handheld device, and a second display that is separated from the handheld device;
(iv) displaying at least one piece of information selected from said n pieces of information on the first display; and
(v) displaying at least one piece of information selected from said n pieces of information on the second display. The two "at least one piece of information" in steps (iv) and (v) may be the same or different.

The above features and advantages and other features and advantages of the present invention are readily apparent from the following detailed description of the best modes for carrying out the invention when taken in connection with the accompanying drawings.

### BRIEF DESCRIPTION OF THE SEVERAL VIEWS OF THE DRAWINGS

The present invention is illustrated by way of example, and not by way of limitation, in the figures of the accompanying drawings and in which like reference numerals refer to similar elements. All the figures are schematic and generally only show parts which are necessary in order to elucidate the invention. For simplicity and clarity of illustration, elements shown in the figures and discussed below have not necessarily been drawn to scale. Well-known structures and devices are shown in simplified form in order to avoid unnecessarily obscuring the present invention. Other parts may be omitted or merely suggested.
Figure 1 schematically shows an operational system or an apparatus for a human operator to operate on a workpiece in accordance with an exemplary embodiment of the present invention.
Figure 2 is a block diagram of a method for using the operational system (or apparatus) as shown in Figure 1 in accordance with an exemplary embodiment of the present invention.
Figure 3 illustrates a dental surgical system or a dental apparatus for a dental surgeon to drill an implant site on a patient's jawbone in accordance with an exemplary embodiment of the present invention.
Figure 4 demonstrates a graph displayed on a dental drill showing the drilling orientation of the drill bit against a preplanned drilling orientation in accordance with an exemplary embodiment of the present invention.
Figure 5 demonstrates a graph displayed on a dental drill showing the drilling depth of a drill bit against a preplanned drilling depth of the drill bit in accordance with an exemplary embodiment of the present invention.
Figure 6 schematically shows a 3D imaging and 3D tracking system in accordance with an exemplary embodiment of the present invention.
Figure 7 schematically shows the approach to display spatial relationships in accordance with an exemplary embodiment of the present invention, where the displayed image is correlated to the world coordinate system, and in other words, the displayed image will not follow the orientation of the dental drill or the display.

### DETAILED DESCRIPTION OF THE PREFERRED EMBODIMENT

In the following description, for the purposes of explanation, numerous specific details are set forth in order to provide a thorough understanding of the present invention. It is apparent, however, to one skilled in the art that the present invention may be practiced without these specific details or with an equivalent arrangement.

Where a numerical range is disclosed herein, unless otherwise specified, such range is continuous, inclusive of both the minimum and maximum values of the range as well as every value between such minimum and maximum values. Still further, where a range refers to integers, only the integers from the minimum value to and including the maximum value of such range are included. In addition, where multiple ranges are provided to describe a feature or characteristic, such ranges can be combined.

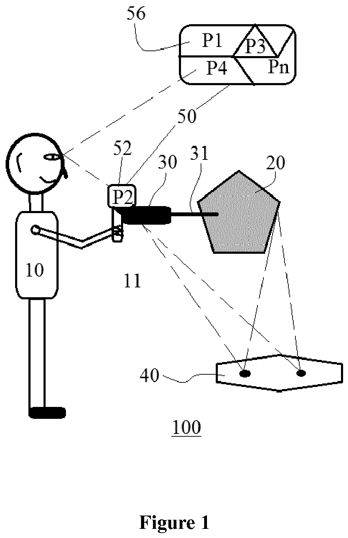

Referring to Figure 1, a human operator 10 uses an operational system (or apparatus) 100 to operate on a workpiece 20. A handheld device 30 is provided for the human operator 10 to hold in his/her hand 11. An action component 31 included in, extended from, or emitted from, handheld device 30 is working on the workpiece 20, under the control of the human operator 10. Action component 30 may be selected from a mechanical part such as drill bit, mill, grinder and blade; an electromagnetic radiation, a laser beam, a liquid flow, a gas stream, and an ultrasound wave etc., or any combination thereof.

Sensing system 40 in Figure 1 may be a three-dimensional (3D) sensing system (e.g. 3D camera, 3D sensor head, or 3D tracking system) that measures the spatial relationship between the action component 31 and the workpiece 20. As will be explained later, this can be accomplished by providing both the action component 31 and the workpiece 20 with trackability by the sensing system 40, coupled with pre-determined 3D information of the action component 31 and the workpiece 20.

A display system 50 is used for displaying n pieces of information P1, P2...Pn which are selected from the working conditions of the handheld device 30, the conditions of the workpiece 20, a real-time spatial relationship between the action component 31 and the workpiece 20, and a preplanned spatial relationship between the action component 31 and the workpiece 20, such as preplanned trajectory of the action component 31 relative to the workpiece 20. Number n is an integer and n≥2. The n pieces of information P1, P2...Pn may be represented as images, symbols, numbers, charts, curves, tables, texts, or any combination thereof.

In Figure 1, the display system 50 comprises a first display 52 that is integrated with the handheld device 30 and a second display 56 that is separated from the handheld device 30. However, it should be appreciated that first display 52 may alternatively be separated from the handheld device 30, and have a shortest distance Dmin1 therebetween of less than 30, 20, 10or 5 centimeters to the handheld device 30. For example, when first display 52 is attached to, or integrated with, the handheld device 30, Dmin1 = 0. As known to skilled person in geometry, handheld device 30 as a 3D object may be considered to consist of m spatial points, and first display 52may be considered to consist of nspatial points, wherein each spatial point may be defined as a conceptual point or preferably a point with a sufficiently small volume (therefore not making m and n infinite numbers). There will be mxn point-to-point distances available, and the smallest value among these mxn point-to-point distances is defined as Dmin1.By the same token, it should be appreciated that second display 56 may have a shortest distance Dmin2 to the handheld device, and Dmin2 is generally greater than any distance between first display 52 and handheld device 30, for example Dmin2 > Dmin1. In some embodiments, shortest distance Dmin2 may be greater than 100 centimeters, greater than 200 centimeters, greater than 300 centimeters, or greater than 400 centimeters.

The first display 52 displays at least one piece of information selected from aforementioned n pieces of information, for example P2. The second display 56 displays at least one piece of information selected from aforementioned n pieces of information, for example, P1, P3, P4... and Pn.

Figure 2 is a block diagram of a method for using the operational system (or apparatus) 100 as shown in Figure 1. At step 210, a human operator holds in his hand a handheld device having an action component, and controls the handheld device so that the action component works on a workpiece according to a predetermined work plan. At step 220, a sensing system measures the real-time spatial relationship between the action component and the workpiece. This can be accomplished by tracking the spatial position and orientation of the action component as well as that of the workpiece. The workpiece may be represented as a previously stored 3D image of the workpiece. At step 230, a display system displays n pieces of information P1, P2...Pn related to the operation. The display system comprises a first display and a second display. The first display has a shortest distance Dmin1 of less than 30 centimeters to the handheld device. For example, when the first display is attached to, or integrated with, the handheld device, Dmin = 0. The first display displays at least one piece of information selected from said n pieces of information. The second display, which is separated from the handheld device, displays at least one piece of information selected from said n pieces of information.

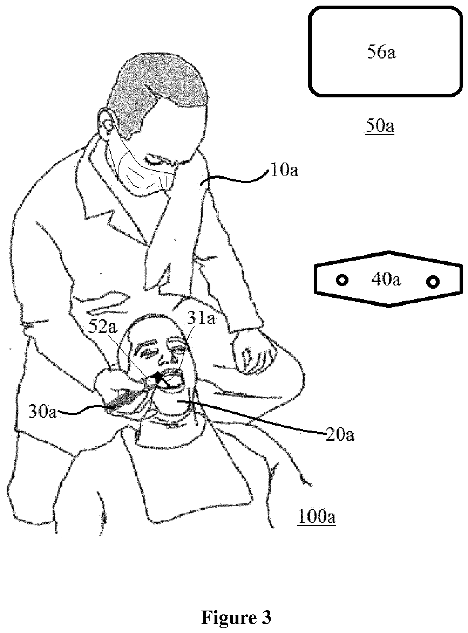

An exemplary embodiment of the invention is illustrated in Figure 3. Referring to Figure 3 in light of Figures 1 and 2, a dental surgical system 100a is an example of the operational system 100 in Figure 1, and is used by a human operator 10 such as a dental surgeon 10a. Dental surgeon 10a is preparing a drilled core for the placement of a dental implant on the workpiece 20 such as a jawbone 20a.The handheld device 30 is a dental drill 30a. The action component 31 of the handheld device 30is exemplified as the drill bit 31a of the dental drill 30a.

In Figure 3, a sensing system 40a measures the spatial relationship between the dental drill 30a and the jawbone 20a. A display system 50a is designed to display n pieces of information P1, P2...Pn which are selected from the working conditions of the dental drill 30a, the conditions of the jawbone 20a, the 3D image of the dental drill 30a, the 3D image of the jawbone 20a, a real-time spatial relationship between the drill bit 31a and the jawbone 20a, and a preplanned spatial relationship between drill bit 31a and the jawbone 20a, wherein n is an integer and n ≥2. The n pieces of information P1, P2...Pn may be represented as images, symbols, numbers, charts, curves, tables, texts, or any combination thereof.

In Figure 3, the display system 50a comprises a first display 52a that is integrated with the dental drill 30a and a second display 56a that is separated from the dental drill 30a. However, it should be appreciated that first display 52a may have a shortest distance Dmin1a of less than 30 centimeters to the handheld device. For example, when first display 52a is attached to, or integrated with, the handheld device 30a, Dmin1a = 0.

In a normal operation, the second display 56a is placed above the head of the surgeon 10a. The first display 52a displays at least one piece of information selected from aforementioned n pieces of information. The second display 56a displays at least one piece of information selected from aforementioned n pieces of information. Said two "at least one piece of information" may be the same or different.

Generally, the size or displaying area of the second display 56a is at least 50 times bigger than the first display 52a. In some embodiments, the size or displaying area of the second display 56a may be at least 100 times, at least 200 times, or even at least 300 times, bigger than the first display 52a. For example, the first display 52a may have a squareshape, a circle shape, or a rectangular shape. The maximum linear dimension of the first display 52a may be in the range of 0.5 inch to 5 inches, such as 0.8 inch to 3 inches, or 1 inch to 2 inches. The shortest distance between the central position of the first display 52a and the tip of the dental drill 30amay be in the range of 0.5 inch to 10 inches, such as 1 inch to 8 inches, or 2 inches to 4 inches.

During the drilling procedure, the surgeon 10a will have to, on one hand, keep an eye on the drilling site of the patient's jawbone for safety concerns, and on another, observe or read the n pieces of information P1, P2...Pn displayed on system 50a. The n pieces of information typically require different observation frequencies. For example, the surgeon may need to read some information pieces every second, while read other information pieces every one minute or every 5 minutes. Say, the surgeon 10a needs to observe the n pieces of information P1, P2...Pn at an observation frequency of F1, F2...and Fn respectively.

In prior art, a surgical room is equipped with only a display like second display 56a, and the dental drill does not have a display like first display 52a. As a result, all the information will be displayed on the second display 56a only. If the surgeon 10a needs to observe both the drilling site on the patient's jawbone and second display 56a, he must keep changing his field of view by moving his head up and down at the highest observation frequency of F1, F2...and Fn. This rigorous requirement makes the surgeon 10a feel nervous and stressful, and increases the likelihood of misoperation, which may result in an irreparable damage on the patient's jawbone or a poor execution of the drilling plan.

In a preferred embodiment according to the present invention, the first display 52a displays at least the piece of information requiring the highest observation frequency. The display 52a is integrated with the dental drill 30a, and is therefore in close proximity to the drilling site on the patient's jawbone. Both the drilling site on the patient's jawbone and first display 52a are within a substantially same field of view of surgeon 10a. Second display 56a is not within said field of view. The field of view (also field of vision) is the extent of the observable world that is seen at any given moment.

If the surgeon 10a needs to observe both the drilling site on the patient's jawbone and first display 52a, he does not need to change his field of view by moving his head up and down at the highest observation frequency of F1, F2...and Fn. The surgeon 10a may or may not need to move his or her eyeballs when monitoring the drilling site and display 52a at the same time. Consequently, the surgeon 10a only needs to change his field of view and move his head up and down to read second display 56a at a much lower frequency. The observation frequency for display 56a in the absence of display 52a may be 2 times or higher, 5 times or higher, or 10 times or higher than that in the presence of display 52a. Technical benefits derived from this feature include ergonomic design, simplicity of operation, improved operational safety, higher productivity, and enhanced efficiency, among others.

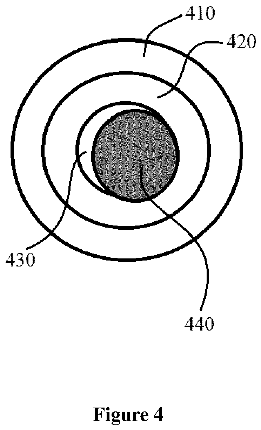

For example, first display 52a may display a graph showing a dynamic or real-time spatial relationship between the drill bit 31a and the jawbone 20a against a predetermined operational plan associated with said spatial relationship for operating on the jawbone 20a.Figure 4 is an exemplary graph on first display 52a showing the drilling orientation of the drill bit 31a against a preplanned drilling orientation. Referring to Figure 4, zone 410 is the safety zone for drilling the jawbone. Within zone 410, an implant is planned at site 420, and accordingly, drilling position is planned at circular area 430. During the surgical operation, the actual position of the drill bit 31a is represented as a circular area 440. When 440 is not within 430, the surgeon may adjust and correct the position of drill 30a, so that 440 is within 430, preferably 440 and 430 are concentric.

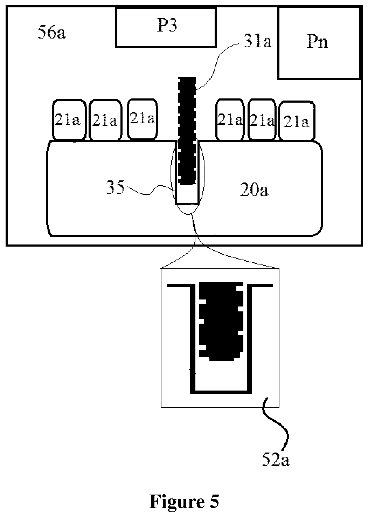

Figure 5 illustrates two exemplary graphs displayed on displays 52a and 56a separately, showing the drilling depth of the drill bit 31a against a preplanned drilling depth of the drill bit 31a. Within display 56a, a drilling depth 35 is preplanned on jawbone 20a, taking into account of many surrounding healthy teeth 21a. The actual position of drill bit 31a is displayed against, or compared to, drilling depth 35 as planned. When the drilling depth reaches the desired value, the surgeon may stop drill bit 31a from drilling any further into the jawbone 20a. A critically important portion of display 56a (in circled area, and demands higher observation frequency) is reproduced and displayed within first display 52a on the dental drill for the convenience of the surgeon 10a. This portion provides a quick reference for the surgeon without the need of any head movement.

The so-called "n pieces of information" should cover a broad range of information, as long as they are related to the operation and they should be delivered to the operator and his assistants. Examples of the n pieces of information may include exact knowledge of the bone topology of the jaw. Such information can be acquired from, for example, computer-generated panoramic and oblique radiographic CT scans of the jaw, which provide the cross-sectional shape of the jaw at every point throughout the arch on a 1:1 scale.

The concept of "spatial relationship" involves analytic geometry or Cartesian geometry that describes every point in three-dimensional space by means of three coordinates. Three coordinate axes are given, each perpendicular to the other two at the origin, the point at which they cross. They are usually labeled x, y, and z. Relative to these axes, the position of any point in three-dimensional space is given by an ordered triple of real numbers, each number giving the distance of that point from the origin measured along the given axis, which is equal to the distance of that point from the plane determined by the other two axes. In addition to Cartesian coordinate system, other coordinate systems may also be used for convenience, for example, cylindrical coordinate system and spherical coordinate system, among others.

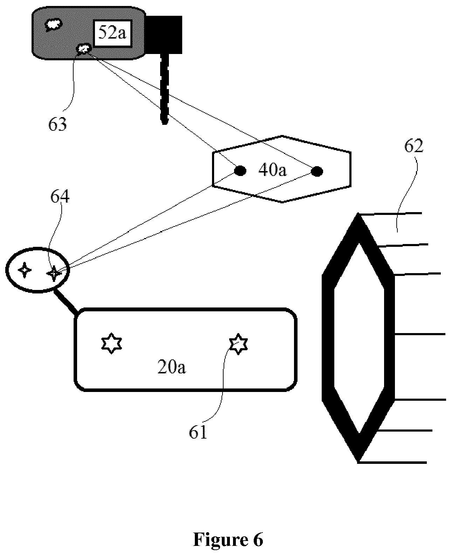

Known techniques for 3D imaging and 3D tracking, if suitable, can be utilized in the present invention, as schematically illustrated in Figure 6. For example, 3D image of jawbone 20a may be obtained using a registration device 61, for acquiring positional determination data of the jawbone. The jawbone can be imaged by any 3D imaging apparatus 62 such as CT or MRI. The registration device 61 contains a suitable material such as a metallic material, which appears clearly on the CT and MRI images. The registration device 61 may be inserted in a reproducible manner into the mouth of the patient at the time the scan is being performed, and its location is registered on the images during the scanning. For example, the registration device 61 may be held in the mouth with a splint attached adhesively to the teeth of the patient by methods known in the dental arts.

The position and orientation of the drill 30a and the drill bit 31a is supplied to the sensing system 40a by means of a first tracking device 63, e.g. LED's attached to the drill body. For example, the drill body or shank may be equipped with a number of LED emitters, whose radiation is tracked by sensing system 40a. The position of these LED's may be tracked by means of a triangulation tracking and measurement technique, or any other suitable tracking and measurement technique, such that the spatial position and orientation of the drill 30a, particularly the drill bit 31a, is known at all times. The term "tracking device" should be understood broadly as including any form of sensor device operative for providing 3-D information about the position of the tracked body such as the drill 30a, drill bit 31a and jawbone 20a.

Similarly, the position and orientation of the jawbone 20a being drilled is also supplied to the sensing system 40a by means of a second tracking device 64 (e.g. LED) whose position is defined relative to the patient's jaw or jawbone. Because of the function of the first and second tracking devices 63 and 64, the real-world positions of the drill 30a, drill bit 31a, the jawbone 20aand related tooth or teeth can be spatially and definitively tracked by the sensing system 40a.

The defined spatial relationship between the second tracking device 64 and the patient's jawbone 20a can be established using any known methods, with or without the use of the registration device 61 in CT or MRI scanning asan "intermediate" reference. If the registration device 61 is not used, then the second tracking device 64 must have a predefined and fixed spatial and angular relationship to the jawbone 20a. If the registration device 61 is used, then the second tracking device 64 can first establish a fixed spatial and angular relationship to the registration device 61, which has a predefined and fixed spatial and angular relationship to the jawbone 20a. A skilled person in the art can then calculate the fixed spatial and angular relationship between the second tracking device 64 and jawbone 20a. This correlation enables the virtual-world CT or MRI scans to be related to the real world jawbone/teeth anatomy, which is trackable via second tracking device 64 in real time by the system 40a.

After the patient is scanned by a CT or MRI imaging system, the data is transferred to the sensing system 40a as the base image display to be used by the dental surgeon in performing the procedure to be undertaken. This CT or MRI image data is correlated by the sensing system 40a with the information generated in real time of the position of the dental drill and of the patient's jawbone, both of which may be constantly changing with movement. The drill 30a position can thus be displayed overlaid onto the images on display system 50a of the patient's jaw and teeth with spatial and angular accuracy. As a result, display system 50a (on 52a, 56a or both) can provide the dental surgeon 10a with a continuous, real-time, three-dimensional image of the location and direction of the drill into the jawbone at all times during the drilling procedure. There should be optimal correlation between the implantation planning and the actual surgical performance, and accurate placement of the insert.

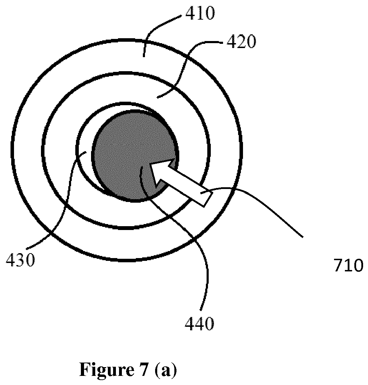

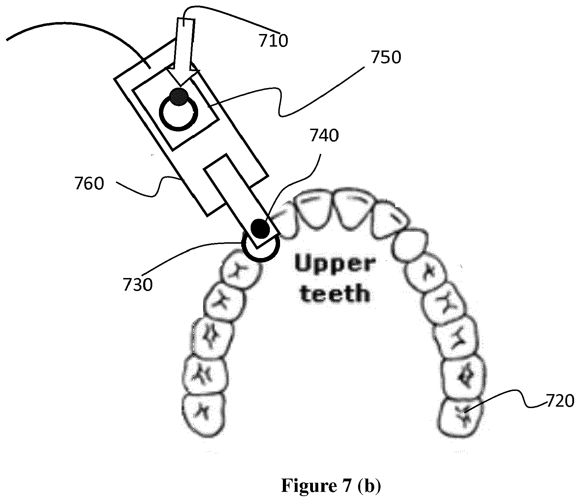

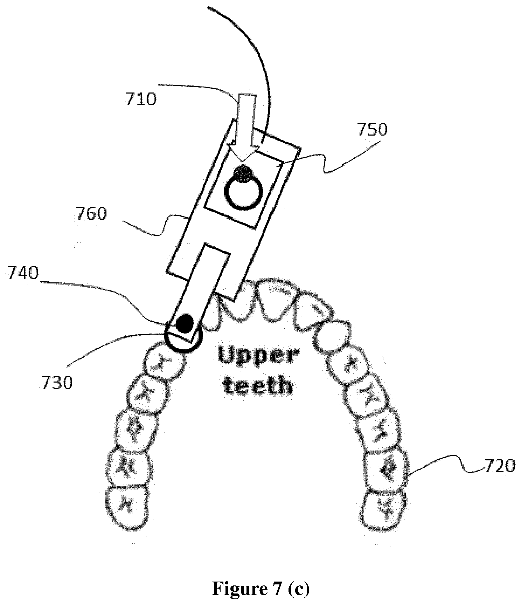

Figure 7 further illustrates the approach to display the space relationships between the drill and the patient's jaw bone. As Figure 4 indicated, that the displayed image provides the operator the feedback how the surgical tools or drills need to be moved. Figure 4 is duplicated as Figure 7(a), and the arrow 710 indicates how the surgeon needs to move the drill.

Figure 7(b) shows the actual scenario with patient teeth 720, the desired position of the drill 730, the actual position of the drill 740, the drilling handpiece, 760 the display 750 on the handpiece. In this illustration it is assumed that the display 750 is integrated onto handpiece 740. In the display 750, the desired drill position and the actual tooth position are displayed corresponding to 730 and 740. Figure 7(c) show same elements of figure 7(b) but the handpiece 760 is in a different orientation, considering that the surgeon may hold the handpiece in a different way. Comparing figure 7(b) and 7(c) one can find that the orientation of the displayed images on display is coresponding to the actual relationships between the handpiece and the patient. When the handpiece is moved from one position/orientation to another, the display must be adjusted. In Figure 7(c), it is rotated. Therefore the displayed information on the second display is rotated so that the desired movement direction of the handpiece can be properly inferred independent from the orientation of the handpiece. Therefore the said display intended for the high frequency information displays the spatial relationships between the handpiece, the drill and the patient. The display orientation remains consistent in the world coordinate system instead of in the coordinate system of the display. Otherwise, if the displayed graph is correlated with the handpiece, when the handpiece rotates from 7(b) position to 7(c) position, the displayed graph will rotate too, therefore the surgeon cannot infer the proper direction to move the handpiece.

Techniques and technologies may be described herein in terms of functional and/or logical block components, and with reference to symbolic representations of operations, processing tasks, and functions that may be performed by various computing components or devices. Such operations, tasks, and functions are sometimes referred to as being computer-executed, computerized, processor-executed, software-implemented, or computer-implemented. It should be appreciated that the various block components shown in the figures may be realized by any number of hardware, software, and/or firmware components configured to perform the specified functions. For example, an embodiment of a system or a component may employ various integrated circuit components, e.g., memory elements, digital signal processing elements, logic elements, look-up tables, or the like, which may carry out a variety of functions under the control of one or more microprocessors or other control devices.

When implemented in software or firmware, various elements of the systems described herein are essentially the code segments or executable instructions that, when executed by one or more processor devices, cause the host computing system to perform the various tasks. In certain embodiments, the program or code segments are stored in a tangible processor-readable medium, which may include any medium that can store or transfer information. Examples of suitable forms of non-transitory and processor-readable media include an electronic circuit, a semiconductor memory device, a ROM, a flash memory, an erasable ROM (EROM), a floppy diskette, a CD-ROM, an optical disk, a hard disk, or the like.

In the foregoing specification, embodiments of the present invention have been described with reference to numerous specific details that may vary from implementation to implementation. The specification and drawings are, accordingly, to be regarded in an illustrative rather than a restrictive sense. The sole and exclusive indicator of the scope of the invention, and what is intended by the applicant to be the scope of the invention, is the literal and equivalent scope of the set of claims that issue from this application, in the specific form in which such claims issue, including any subsequent correction.

## Claims

1. An operational system for a human operator to operate on a workpiece comprising:
a handheld device for the human operator to hold in hand, wherein the handheld device includes an action component that works on the workpiece under the control of the human operator;
a sensing system that measures the spatial relationship between the action component and the workpiece;
a display system for displaying n pieces of information P1, P2...Pn which are selected from the working conditions of the handheld device, the conditions of the workpiece, the 3D image of the handheld device, the 3D image of the workpiece, a real-time spatial relationship between the action component and the workpiece, and a preplanned spatial relationship between the action component and the workpiece, wherein n is an integer and n ≥2;
wherein the display system comprises a first display that has a shortest distance Dmin1 of less than 30 centimeters to the handheld device; and a second display that is separated from the handheld device;
wherein the first display displays at least one piece of information selected from said n pieces of information; and
wherein the second display displays at least one piece of information selected from said n pieces of information.

2. The operational system according to Claim 1, wherein the action component is selected from a mechanical part such as a drill bit, a mill, a grinder and a blade; an electromagnetic radiation, a laser beam, a liquid flow, a gas stream, and an ultrasound wave.

3. The operational system according to Claim 1, wherein said n pieces of information P1, P2...Pn are represented as images, symbols, numbers, charts, curves, tables, texts, or any combination thereof.

4. The operational system according to Claim 1, wherein the first display is a virtual display.

5. The operational system according to Claim 4, wherein the operational system is a dental surgical system, the first display is integrated with the handheld device, and the handheld device is a dental drill.

6. The operational system according to Claim 5, wherein the action component of the handheld device is the drill bit of the dental drill.

7. The operational system according to Claim 6, wherein the human operator needs to observe the n pieces of information P1, P2...Pn at an observation frequency of F1, F2...and Fn respectively; and
wherein the first display displays at least the piece of information requiring the highest observation frequency.

8. The operational system according to Claim 7, wherein said piece of information requiring the highest observation frequency is a graph showing a dynamic spatial relationship between the drill bit and the jawbone against a predetermined operational plan associated with said spatial relationship for operating on the jawbone.

9. The operational system according to Claim 8, wherein said graph shown in said first display is shown in a world coordinate system independent of the coordinate system of the display device, whereby when said action component and/or the first display is rotated from one orientation to another, the orientation of the displayed graph remains the same in the coordinate system.

10. The operational system according to Claim 8, wherein said piece of information requiring the highest observation frequency is a graph showing the drilling orientation of the drill bit against a preplanned drilling orientation, or is a graph showing the drilling depth of the drill bit against a preplanned drilling depth of the drill bit.

11. A method of operating on a workpiece comprising:
(i) providing a handheld device for a human operator to hold in hand, wherein the handheld device includes an action component that works on the workpiece under the control of the human operator;
(ii) measuring the spatial relationship between the action component and the workpiece with a sensing system;
(iii) providing a display system for displaying n pieces of information P1, P2...Pn which are selected from the working conditions of the handheld device, the conditions of the workpiece, the 3D image of the handheld device, the 3D image of the workpiece, a real-time spatial relationship between the action component and the workpiece, and a preplanned spatial relationship between the action component and the workpiece, wherein n is an integer and n ≥2; and wherein the display system comprises a first display that has a shortest distance Dmin1 of less than 30 centimeters to the handheld device; and a second display that is separated from the handheld device;
(iv) displaying at least one piece of information selected from said n pieces of information on the first display; and
(v) displaying at least one piece of information selected from said n pieces of information on the second display.

12. The method according to Claim 11, wherein the action component is selected from a mechanical part such as a drill bit, a mill, a grinder and a blade; an electromagnetic radiation, a laser beam, a liquid flow, a gas stream, and an ultrasound wave.

13. The method according to Claim 11, wherein said n pieces of information P1, P2...Pn are represented as images, symbols, numbers, charts, curves, tables, texts, or any combination thereof.

14. The method according to Claim 11, wherein the handheld device is a dental drill in a dental surgical system; and the action component of the handheld device is the drill bit of the dental drill.

15. The method according to Claim 14, wherein the workpiece is a jawbone; and the 3D image of the jawbone is a CT or MRI image.

16. The method according to Claim 15, wherein the human operator needs to observe the n pieces of information P1, P2...Pn at an observation frequency of F1, F2...and Fn respectively; and
wherein the first display displays at least the piece of information requiring the highest observation frequency.

17. The method according to Claim 16, wherein said piece of information requiring the highest observation frequency is a graph showing a dynamic spatial relationship between the drill bit and the jawbone against a predetermined operational plan associated with said spatial relationship for operating on the jawbone.

18. The method according to Claim 16, wherein said piece of information requiring the highest observation frequency is a graph showing the drilling orientation of the drill bit against a preplanned drilling orientation, or is a graph showing the drilling depth of the drill bit against a preplanned drilling depth of the drill bit.
